# EUROPEAN PATENT SPECIFICATION

(11) **EP 2 154 528 B1**
(45) Date of publication and mention of the grant of the patent: **24.01.2018**
(21) Application number: 08776948.5
(22) Date of filing: 28.05.2008
(51) Int. Cl.: G01N 31/00, G01N 21/77, G01N 31/10, G01N 31/22

(54) **HYDROGEN SENSOR**
WASSERSTOFFSENSOR
DÉTECTEUR D'HYDROGÈNE

(30) Priority: 04.06.2007 JP 2007148001
(43) Date of publication of application: 17.02.2010
(73) Proprietor: Kabushiki Kaisha Atsumitec, Shizuoka 431-0192 (JP); National Institute of Advanced Industrial Science and Technology, Tokyo 100-8921 (JP)
(72) Inventor: UCHIYAMA, Naoki, Hamamatsu-shi Shizuoka 431-0192 (JP); YOSHIMURA, Kazuki, Nagoya-shi Aichi 463-8560 (JP)
(74) Representative: advotec.
(86) International application number: PCT/JP2008/059833
(87) International publication number: WO 2008/149752

(56) References cited:
- EP-A1- 1 775 578
- WO-A1-2007/049965
- WO-A1-2007/108261
- WO-A1-2007/108276
- JP-A- 02 165 034
- JP-A- 2003 147 463
- JP-A- 2003 147 463
- JP-A- 2005 083 832
- BAO S ET AL: "Titanium-buffer-layer-inserted switchable mirror based on MgNi alloy thin film", JAPANESE JOURNAL OF APPLIED PHYSICS, JAPAN SOCIETY OF APPLIED PHYSICS, JP, vol. 45, no. 23, 1 January 2006 (2006-01-01), pages L588-L590, XP008122483, ISSN: 0021-4922, DOI: 10.1143/JJAP.45.L588 [retrieved on 2006-06-09]
- PASTUREL M ET AL: "Stabilized switchable black state in Mg2NiH4/Ti/Pd thin films for optical hydrogen sensing", APPLIED PHYSICS LETTERS, A I P PUBLISHING LLC, US, vol. 89, no. 2, 12 July 2006 (2006-07-12), pages 21913-021913, XP012086944, ISSN: 0003-6951, DOI: 10.1063/1.2221412
- BAO S. ET AL.: 'Titanium-buffer-layer-inserted switchable mirror based on MgNi alloy thin film' JPN. J. APPL. PHYS. vol. 45, no. 23, 2006, pages L588 - L590, XP008122483
- PASTUREL M. ET AL.: 'Stabilized switchable "black state" in Mg2NiH4/Ti/Pd thin films for optical hydrogen sensing' APPL. PHYS. LETT. vol. 89, no. 2, 2006, pages 021913-1 - 021913-3, XP012086944
- YOSHIMURA K.: 'Gas Chromism (Gas Chromic Glass)' OPTICAL AND ELECTRO-OPTICAL ENGINEERING CONTACT vol. 45, no. 10, 2007, pages 516 - 520, XP008122488

## Description

### Technical Field

The present invention relates to a hydrogen sensor for detecting hydrogen gas.

### Background Art

From a viewpoint of preventing carbon dioxide emissions into the atmosphere, hydrogen has been attracting attention as an energy source. There is, however, a risk of explosion if hydrogen gas leaks into an atmosphere. Thus, the development of a hydrogen sensor capable of quickly detecting leaked hydrogen gas has been being advanced. As such hydrogen sensor, a semiconductor sensor using tin oxide has been developed. The operating temperature of this semiconductor sensor is, however, as high as about 400°C. Thus, in using this semiconductor sensor, it is necessary to take a preventive measure against explosion. Consequently, a hydrogen gas leak detector using this semiconductor sensor has a drawback that it is complicated in structure.

In this situation, there have been developed hydrogen sensors in which a thin film layer of a magnesium-nickel alloy or the like formed on the surface of a substrate of glass or the like is quickly hydrogenated in the presence of hydrogen gas under the action of a catalyst layer of palladium or the like, and thus undergoes changes in material properties. A hydrogen sensor of this type is disclosed in Unexamined Japanese Patent Publication No. 2005-83832 (hereinafter referred to as Patent Document 1), for example. The hydrogen sensor disclosed in Patent Document 1 can detect hydrogen gas that has leaked into an atmosphere by detecting a change in optical reflectance (hereinafter, sometimes referred to simply as "reflectance") of the thin film layer caused by hydrogenation. The hydrogen sensor disclosed in Patent Document 1, in which the thin film layer is reversibly hydrogenated at normal temperatures, has also an advantage that it can detect leaked hydrogen gas safely and quickly.

In the hydrogen sensor disclosed in Patent Document 1, however, the catalyst layer, which is directly exposed to the atmosphere, is prone to oxidation, because magnesium, which is a constituent of the thin film layer, diffuses, deposits or the like (hereinafter, sometimes the word "diffuse" is used to cover this meaning) in the catalyst layer, as the hydrogenation and dehydrogenation are repeated (hereinafter, wording "repetition of hydrogenation and dehydrogenation" is sometimes simplified into wording "repetition of hydrogenation"). Thus, there is a risk that the oxidation of the catalyst layer entails oxidation of the thin film layer, resulting in a smaller change in reflectance, and therefore, a decrease in leaked hydrogen gas detection sensitivity of the thin film layer (which means deterioration of the hydrogen sensor).

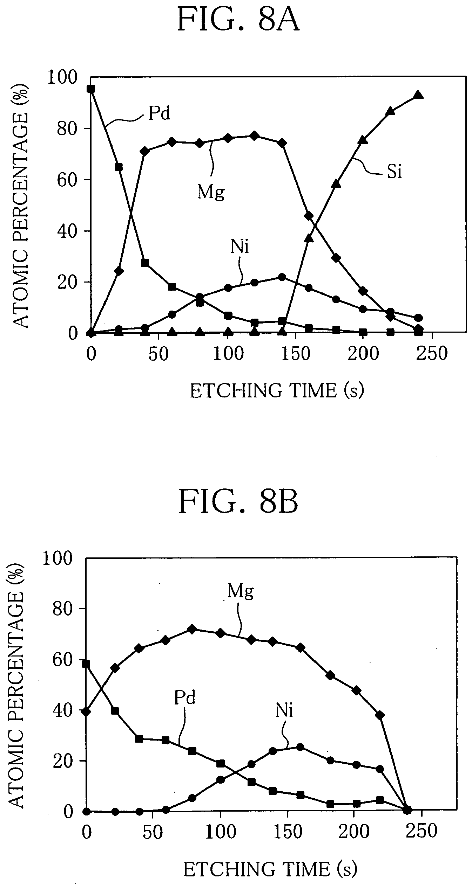

FIGS. 8A and 8B are diagrams for explaining an example of deterioration of a hydrogen sensor including a thin film layer of a magnesium-nickel alloy and a catalyst layer of palladium formed over a glass substrate, obtained by XPS (X-ray photoelectron spectroscopy). In the diagrams, etching time (in sec) plotted on the horizontal axis corresponds to depth from the surface of the palladium catalyst layer, and the etching time of 250 sec corresponds to the depth of about 50nm (the origin, or zero corresponds to the surface of the palladium catalyst layer). On the
vertical axis, atomic percentage (hereinafter, sometimes the symbol "%" is used in place of this term) is plotted.

FIG. 8A shows a result of measurement on the hydrogen sensor before undergoing repetition of hydrogenation. FIG. 8A shows that at the surface of the catalyst layer, palladium (Pd) is present at about 98%, and magnesium (Mg) and nickel (Ni) are at about 0%, respectively.

Incidentally, silicon (Si) is contained in glass constituting the substrate, and the other substances are omitted from the diagrams. Nickel (Ni) is roughly at 0% over a range of etching times from 0 to 40 sec.

FIG. 8B shows a result of measurement on the hydrogen sensor after undergoing repetition of hydrogenation. FIG. 8B shows that at the surface of the catalyst layer, palladium (Pd) has reduced to slightly less than 60%, while magnesium (Mg) has increased to slightly less than 40%. Nickel (Ni) is roughly at 0% over a range of etching times from 0 to 60 sec, which means that in this range, magnesium (Mg) cannot combine with nickel (Ni) by inter-atomic bonding or the like, for example.

Thus, from the result of measurement shown in FIG. 8B, it is recognized that repetition of hydrogenation in the hydrogen sensor changed the material properties of the catalyst layer and the thin film layer, that magnesium (Mg) was diffused in the catalyst layer and thereby the catalyst layer is prone to oxidation, and that reduction in palladium (Pd) in the catalyst layer resulted in degraded catalysis. Incidentally, silicon (Si) is omitted from the diagram of FIG. 8B.

BAO, S., et al., Titanium-buffer-layer-inserted switchable mirror based on MgNi alloy thin film, Jpn. J. Appl. Phys.,2006, Vol. 45, No. 23, pages L588-L59O discloses a mirror comprising a substrate; a thin film layer formed over the substrate; a buffer layer formed over the thin film layer; and a catalyst layer formed over the buffer layer, which, by being contacted by hydrogen gas in an atmosphere, hydrogenates the thin film layer, thereby changing optical reflectance of the thin film layer.

JP 2003-147463 A describes a hydrogen detection apparatus using a hydrogen reaction device. The hydrogen reaction device includes a thin film layer of a hydrogen storage material and a catalyst layer of a material having a high hydrogen dissociation capacity, stacked on a substrate.

### Disclosure of the Invention

In order to solve the above-mentioned problem, the present invention provides a hydrogen sensor having the features of claim 1.

In other words, the characteristic feature of this hydrogen sensor is that between the catalyst layer and the thin film layer, there is formed a buffer layer containing a constituent that combines with a constituent of the thin film layer which diffuses from the thin film layer into the catalyst layer. Thus, in this hydrogen sensor, the constituent of the thin film layer diffuses into the buffer layer and further diffuses into the catalyst layer, together with the constituent of the buffer layer, and both constituents combine with each other by inter-atomic bonding or the like, for example, within the catalyst layer, at the surface of the catalyst layer and elsewhere. The elements combined in this manner are less prone to oxidation than uncombined elements. Thus, the oxidation of the catalyst layer after repetition of hydrogenation is restrained, so that the oxidation of the thin film layer is also restrained. Here, the buffer layer may be formed of either a single constituent (one metal element or the like, for example) or a plurality of constituents (an alloy, for example).

Specifically, the thin film layer may be formed of a magnesium alloy or magnesium, for example, while the catalyst layer may be formed to contain palladium or platinum, for example. The thin film layer formed of such substance can undergo a change in optical reflectance by reversible hydrogenation. The catalyst layer may be formed of any of palladium, platinum, a palladium alloy and a platinum alloy.

More specifically, the thin film layer may be formed of a magnesium-nickel alloy, a magnesium-titanium alloy, a magnesium-niobium alloy, a magnesium-cobalt alloy or a magnesium-manganese alloy, for example. The thin film layer formed of such substance can more quickly undergo reversible hydrogenation.

When the thin film layer is formed of a magnesium alloy or magnesium, the buffer layer is formed to contain a constituent that combines with magnesium that diffuses from the thin film layer into the catalyst layer, thereby restraining the oxidation of the catalyst layer attributed to the magnesium.

In this case, the buffer layer may, specifically, be formed to contain nickel, titanium, niobium or vanadium, for example.

For an improvement in hydrogen gas detection sensitivity, it is desirable that a constituent of the catalyst layer be diffused in the thin film layer. There is however a possibility that, while preventing the oxidation of the catalyst layer as mentioned above, the buffer layer prevents the constituent of the catalyst layer from diffusing into the thin film layer, resulting in the thin film layer difficult to hydrogenate.

However, if, in the above hydrogen sensor, the thickness of the buffer layer is in a preferable range of 1 to 5nm, such phenomenon hardly occurs while the oxidation of the catalyst layer is restrained by the buffer layer.

Preferably, in the above hydrogen sensor, as another preferable measure against the above phenomenon, a thin film activation layer may be interposed between the substrate and the thin film layer and/or between the buffer layer and the thin film layer. The thin film activation layer contains a constituent which, by being contacted by hydrogen, hydrogenates the thin film layer, thereby changing the optical reflectance of the thin film layer.

In the hydrogen sensor structured as described above, even if the buffer layer prevents the constituent of the catalyst layer from diffusing in the thin film layer, the above constituent of the thin film activation layer diffuses in the thin film layer and promotes the hydrogenation and dehydrogenation of the thin film layer, resulting in a further improved hydrogen detection sensitivity of the hydrogen sensor. The thin film activation layer may be formed of either a single constituent (one metal element or the like, for example) or a plurality of constituents (an alloy, for example), as long as it contains a constituent which, by being contacted by hydrogen, hydrogenates the thin film layer, thereby changing the optical reflectance of the thin film layer.

Specifically, in the above hydrogen sensor, the thin film activation layer may be formed to contain the same constituent as the catalyst layer contains. In this case, the constituent having the same catalytic function as the catalyst layer diffuses from the thin film activation layer into the thin film layer, and promotes the hydrogenation and dehydrogenation of the thin film layer. Consequently, desirable hydrogen gas detection sensitivity is maintained, while the oxidation of the catalyst layer is restrained by the buffer layer.

More specifically, in the above hydrogen sensor, the thin film activation layer may be formed to contain
palladium or platinum. In this case, palladium or platinum having a catalytic function diffuses from the thin film activation layer into the thin film layer. Consequently, desirable hydrogen gas detection sensitivity is maintained, while the oxidation of the catalyst layer is restrained by the buffer layer.

### Brief Description of the Drawings

FIG. 1 is a diagram showing schematic cross-sectional structure of a hydrogen sensor according to an example not covered by the claims;
FIG. 2A is a graph showing a result of XPS measurement on the hydrogen sensor of FIG. 1 before undergoing repetition of hydrogenation;
FIG. 2B is a graph showing a result of XPS measurement on the hydrogen sensor of FIG. 1 after undergoing hydrogenation 60 times;
FIG. 3A is a graph showing a result of measurement on change in transmittance of a hydrogen sensor including no buffer layer, caused by repetition of hydrogenation;
FIG. 3B is a graph showing a result of measurement on change in transmittance of a hydrogen sensor including a buffer layer, caused by repetition of hydrogenation;
FIG. 4A is a graph showing a result of XPS measurement on a hydrogen sensor including no buffer layer;
FIG. 4B is a.graph showing a result of XPS measurement on a hydrogen sensor including a buffer layer;
FIG. 5 is a diagram showing schematic cross-sectional structure of a hydrogen sensor according to an example not covered by the claims;
FIG. 6 is a diagram showing schematic cross-sectional structure of a hydrogen sensor as a first variation of the hydrogen sensor shown in FIG. 5;
FIG. 7 is a diagram showing schematic cross-sectional structure of a hydrogen sensor as a second variation of the hydrogen sensor shown in FIG. 5;
FIG. 8A is a graph showing a result of XPS measurement on a conventional hydrogen sensor before undergoing repetition of hydrogenation; and
FIG. 8B is a graph showing a result of XPS measurement on the conventional hydrogen sensor after undergoing repetition of hydrogenation.

### Best Mode of Carrying out the Invention

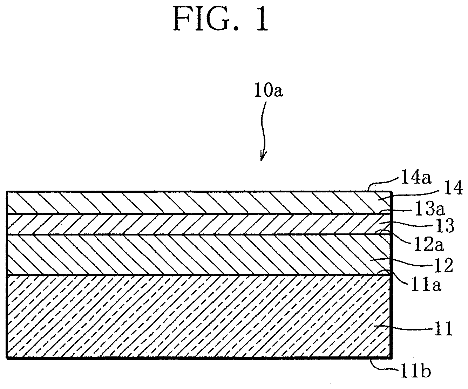

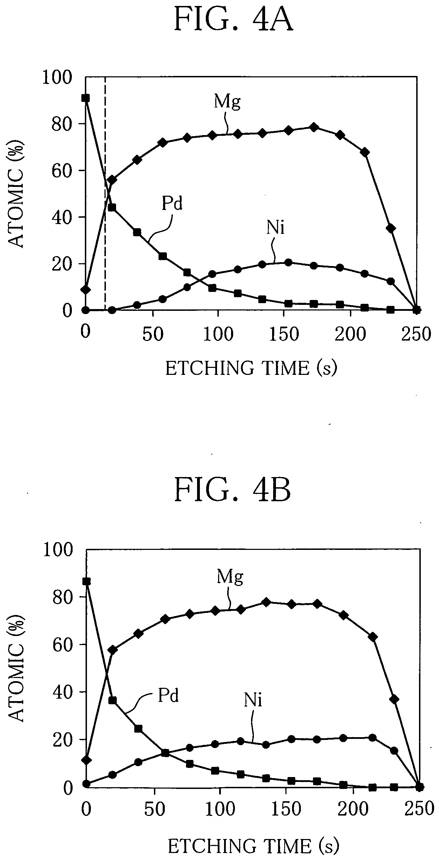

First, referring to FIGS. 1 to 4B, a hydrogen sensor will be described. The hydrogen sensor 10a shown in FIG. 1 includes a substrate 11 of glass. On the surface 11a of the substrate 11, a thin film layer 12 of elemental composition MgNix (0≤x<0.6) is formed. On the surface 12a of the thin film layer 12, a buffer layer 13 of titanium (Ti) is formed. Further, on the surface 13a of the buffer layer 13, a catalyst layer 14 of palladium (Pd) is formed. Here, the thickness of the thin film layer 12 is 40nm, the thickness of the buffer layer 13 is 2nm, and the thickness of the catalyst layer 14 is 4nm.

The buffer layer 13 less than 1nm in thickness results in a reduction in the amount of titanium (Ti) or the like diffusing from the buffer layer 13 into the catalyst layer 14, and therefore difficulty in preventing the oxidation of the catalyst layer 14. The buffer layer 13 more than 5nm in thickness, on the other hand, makes it difficult for a constituent of the catalyst layer 14, such as palladium (Pd), to diffuse into the thin film layer 12, and therefore makes hydrogenation of the thin film layer 12 difficult, which possibly results in a decrease in hydrogen gas detection sensitivity. In the hydrogen sensor 10a, the thickness of the buffer layer 13 is therefore determined by taking account of the balance between the beneficial and adverse effects of the buffer layer 13, i.e., prevention of oxidation of the catalyst layer 14 and decrease in hydrogen gas detection sensitivity.

The buffer layer 13 may be formed of a substance capable of combining with magnesium (Mg) that diffuses from the thin film layer 12 into the catalyst layer 14 to thereby restrain the oxidation of the catalyst layer 14 attributed to the magnesium (Mg). For example, the buffer layer 13 may be formed of nickel, niobium, vanadium or the like. Preferably, the thickness of the catalyst layer 14 may be within a range of 1nm to 100nm.

The thin film layer 12, the buffer layer 13 and the catalyst layer 14 can each be formed by sputtering, vacuum evaporation, electron beam evaporation, plating or the like. The substrate 11 may be an acrylic resin sheet, a polyethylene sheet (polyethylene film) or the like.

When exposed to an atmosphere with a hydrogen concentration of about 100ppm to 1% or more, the hydrogen sensor 10a structured as described above exhibits a visible (visualizable) change in optical reflectance of the thin film layer 12, quickly, namely in several to ten sec or so.

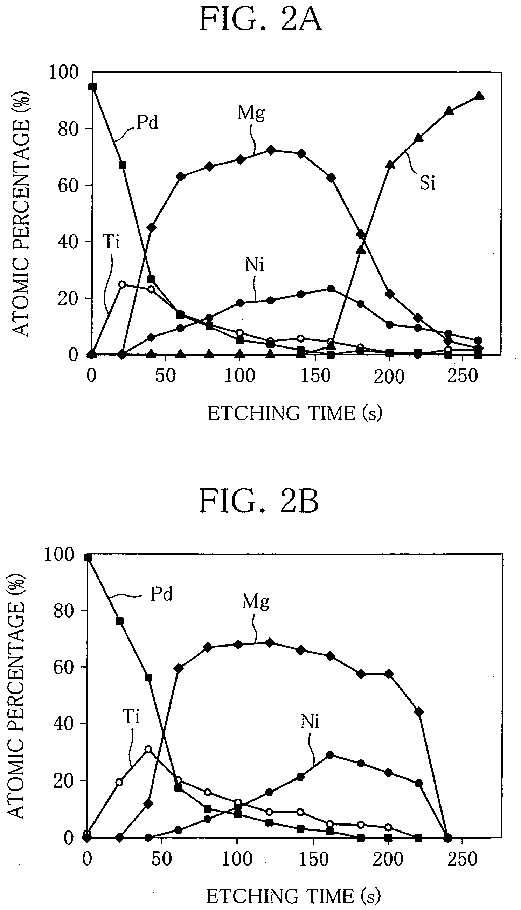

FIG. 2A is a graph showing a result of XPS measurement on the hydrogen sensor 10a before undergoing repetition of hydrogenation, and FIG. 2B is a graph showing a result of XPS measurement on the hydrogen sensor 10a after undergoing hydrogenation 60 times. As the measurement conditions, interval of hydrogenation is set to 5 minutes, and ambient temperature is set to 25°C.

FIG. 2A shows that at the surface 14a of the catalyst layer 14 (etching time 0 sec), palladium (Pd) is present at about 95%, and nickel (Ni) and magnesium (Mg) are at 0%, respectively. Magnesium (Mg) is at about 0% over a range of etching times from 0 to about 20 sec, and increases to slightly more than 60% at the etching time about 60 sec. Palladium (Pd) reduces to about 20% at the etching time 50 sec, and then gently reduces to 0% at the etching time about 160 sec. Since the etching time 250 sec corresponds to the depth of about 50nm, the etching time 20 sec corresponds to the depth of about 4nm.

FIG. 2B shows that at the surface 14a of the catalyst layer 14, palladium (Pd) is present at about 99%, and nickel (Ni) and magnesium (Mg) are at 0%, respectively. Magnesium (Mg) is at about 0% over a range of etching times from 0 to about 20 sec, and increases to slightly more than 60% at the etching time about 60 sec. Palladium (Pd) reduces to about 20% at the etching time about 60 sec, and then gently reduces to 0% at the etching time about 180 sec.

Thus, the diagram shows that in spite of 60 times of hydrogenation, magnesium (Mg) constituting the thin film layer 12 hardly diffuses to the catalyst layer 14, so that very little magnesium (Mg) is present in the region of etching times from 0 to about 20 sec. This means that the oxidation of the catalyst layer 14 can be prevented. Both before and after the repetition of hydrogenation, the maximum atomic percentage of titanium (Ti) is present in the region of etching times from 25 to about 50 sec, which means that the buffer layer 13 is stable. Further, both before and after the repetition of hydrogenation, magnesium (Mg) increases from the etching time about 20 sec, which means that the thin film layer 13 is rather stable. Palladium (Pd) in the catalyst layer 14 does not exhibit a remarkable change, which means the catalyst layer 14 is also stable.

Thus, it is recognized that, in the hydrogen sensor 10a, in spite of repetition of hydrogenation, the buffer layer 13 prevents the oxidation of the catalyst layer 14, and therefore, prevents a decrease in hydrogen detection sensitivity caused by the oxidation of the catalyst layer 14. Incidentally, Si in FIG. 2A is silicon contained in glass constituting the substrate 11. Silicon (Si) is omitted from the diagram of FIG. 2B.

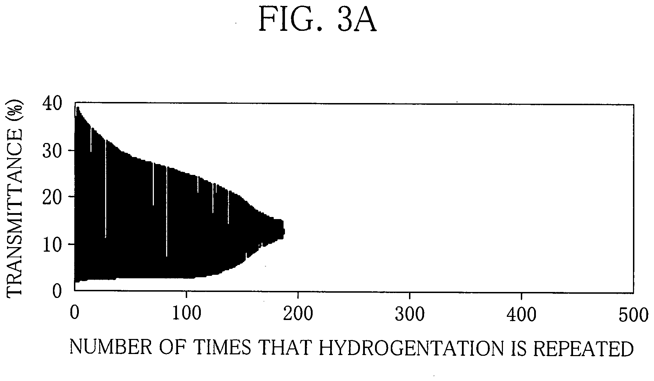

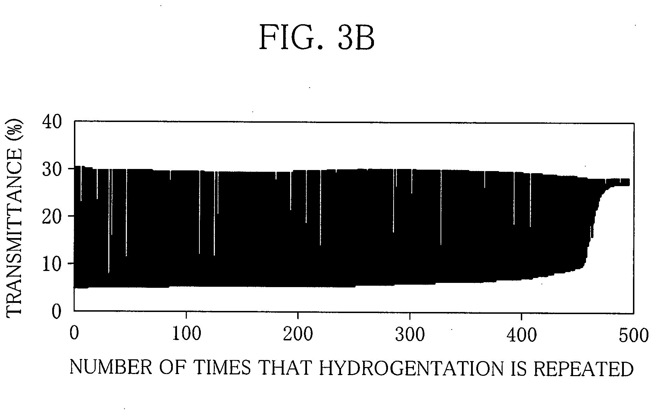

FIG. 3A is a graph showing change in transmittance caused by repetition of hydrogenation, or in other words, deterioration in hydrogen detection sensitivity, for a hydrogen sensor including no buffer layer 13. FIG. 3B, on the other hand, is a graph showing change in transmittance caused by repetition of hydrogenation, or deterioration in hydrogen detection sensitivity, for the hydrogen sensor 10a including the buffer layer 13. Here, the number of times that hydrogenation is repeated is plotted on the horizontal axis, while the transmittance of the hydrogen sensor 10a is plotted on the vertical axis. The transmittance of the hydrogen sensor 10a is, for example the ratio of the amount of light exiting from the catalyst layer 14 to the amount of light falling on the rear side 11b of the substrate 11 at right angles, expressed in percentage.

The hydrogen sensor with the thin film layer not hydrogenated (dehydrogenated) exhibits low transmittance (thus high reflectance), and the transmittance increases (reflectance decreases) with hydrogenation of the thin film layer. Since the width of variation of transmittance with variation of hydrogen gas concentration (hereinafter, sometimes referred to simply as "variation width of transmittance") determines the hydrogen detection sensitivity of the hydrogen sensor, it is desired that the variation width of transmittance stay constant. Further, for stable detection of leaked hydrogen gas present at low concentrations, it is desired that the transmittance with the thin film layer not hydrogenated stay constant. Thus, in the hydrogen sensor, it is desired that the variation width of transmittance as well as the transmittance with the thin film layer not hydrogenated stay constant.

As FIG. 3A shows, in the hydrogen sensor including no buffer layer 13, as the hydrogenation is repeated, the transmittance with the thin film layer hydrogenated decreases and the variation width of transmittance also decreases. After the repetition of hydrogenation reaches 130 times or so, also the transmittance with the thin film layer not hydrogenated changes.

In contrast, as FIG. 3B shows, in the hydrogen sensor 10a including the buffer layer 13, until the repetition of hydrogenation reaches about 450 times, the transmittance with the thin film layer 12 hydrogenated, the variation width of transmittance, and the transmittance with the thin film layer 12 not hydrogenated all change little. Thus, compared with the hydrogen sensor including no buffer layer 13, the hydrogen sensor 10a undergoes only a very small decrease in hydrogen detection sensitivity and can detect leaked hydrogen gas stably. Thus it can be said that it is a hydrogen sensor with extremely high durability and detection sensitivity.

FIG. 4A is a graph showing a result of XPS measurement on a hydrogen sensor including no buffer layer 13 before undergoing repetition of hydrogenation. FIG. 4B is a graph showing a result of XPS measurement on a hydrogen sensor including a buffer layer 13 of nickel (Ni), in place of titanium (Ti) in the hydrogen sensor 10 of FIG. 1, before undergoing repetition of hydrogenation.

As seen in FIG. 4A, nickel (Ni) is at almost 0% over a range of etching times from 0 to 25 sec. This means that in the hydrogen sensor including no buffer layer 13, only magnesium contained in the thin film layer 12 diffuses into the catalyst layer 14 and nickel (Ni) does not diffuse toward the surface 14a of the catalyst layer 14. Consequently, it can be said that the catalyst layer 14 is oxidized as magnesium (Mg) in the catalyst layer 14, not able to combine with nickel (Ni), is oxidized.

In contrast, as seen in FIG. 4B, in the hydrogen sensor including the buffer layer 13 of nickel (Ni), nickel (Ni) is present at about 2% at the etching time 0 sec, and at about 7% at the etching time about 25 sec. This means that nickel (Ni) has diffused from the thin film layer 12 into the catalyst layer 14. Consequently, even when the repetition of hydrogenation causes magnesium (Mg) contained in the thin film layer 12 to diffuse into the catalyst layer 14, magnesium (Mg) diffused in the catalyst layer 14 can combine with nickel (Ni), so that oxidation of the catalyst layer 14 is prevented. Thus, also the buffer layer 13 formed of nickel (Ni) results in improved durability of the hydrogen sensor 10a.

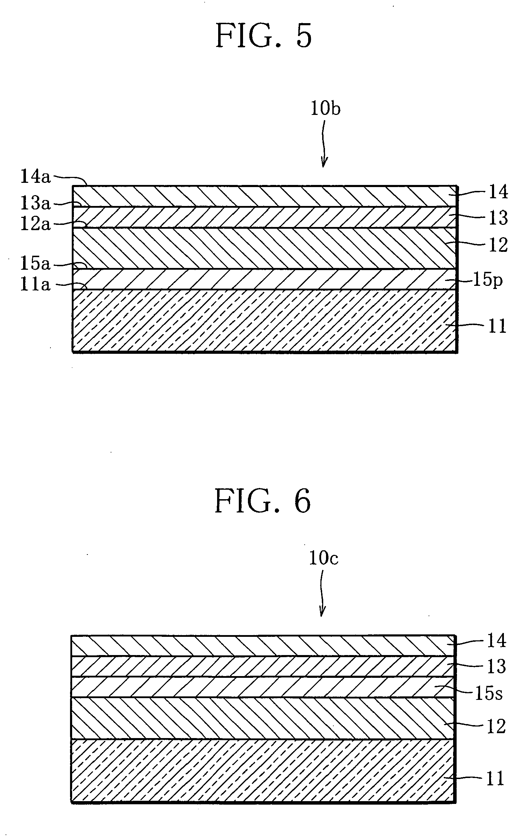

Next, referring to FIG. 5, another example of a hydrogen sensor will be described in detail, where constructional elements similar in function to those of the first example will be assigned the same reference marks, and the description of those constructional elements will be omitted.

In the hydrogen sensor 10b shown in FIG. 5, a first thin film activation layer 15p is formed on the surface 11a of a substrate 11, and a thin film layer 12 is formed on the surface 15a of the first thin film activation layer 15p. Further, a buffer layer 13 is formed on the surface 12a of the thin film layer 12, and a catalyst layer 14 is formed on the surface 13a of the buffer layer 13. In other words, the hydrogen sensor 10b is formed by further interposing a first thin film activation layer 15p between the substrate 11 and the thin film layer 12 in the structure of the hydrogen sensor 10a according to the first example.

The substrate 11, the thin film layer 12, the buffer layer 13 and the catalyst layer 14 of the hydrogen sensor 10b are similar in elemental composition as well as formation to those of the hydrogen sensor 10a according to the first example. The first thin film activation layer 15p contains palladium (Pd) that can act as a catalyst in the catalyst layer 14. Like the hydrogen sensor 10a, the hydrogen sensor 10b includes the buffer layer 13, which can prevent the oxidation of the catalyst layer 14 and the thin film layer 12, thereby preventing a decrease in hydrogen detection sensitivity. In the hydrogen sensor 10b, further, palladium (Pd) diffuses from the first thin film activation layer 15p interposed between the substrate 11 and the thin film layer 12 into the thin film layer 12 and promotes hydrogenation/dehydrogenation of the thin film layer 12. This leads to an improvement in hydrogen detection sensitivity of the hydrogen sensor 10b. Thus, in the hydrogen sensor 10b, while the buffer layer 13 prevents the oxidation of the catalyst layer 14 and the thin film layer 12, the first thin film activation layer 15p compensates for the decrease in hydrogen detection sensitivity attributed to the presence of the buffer layer 13.

In the present example, the thickness of the first thin film activation layer 15p is 2nm. The first thin film activation layer 15p is however provided to make palladium (Pd) diffuse into the thin film layer 12, thereby promoting the hydrogenation of the thin film layer 12. Thus, as long as this object can be achieved, the thickness of the first thin film activation layer 15p is not restricted to that in the present example. Further, since the first thin film activation layer 15p is provided to make a metal acting as a catalyst diffuse into the thin film layer 12, the constituent of the first thin film activation layer 15p is not restricted to palladium (Pd).

Next, referring to FIG. 6, a hydrogen sensor according to the present invention will be described in detail, where constructional elements similar in function to those of the preceding embodiments will be assigned the same reference marks, while the description of those constructional elements will be omitted.

The hydrogen sensor 10c shown in FIG. 6 is formed by further interposing a second thin film activation layer 15s between the buffer layer 13 and the thin film layer 12 in the structure of the hydrogen sensor 10a according to the first embodiment. The second thin film activation layer 15s is formed to contain palladium (Pd). In the hydrogen sensor 10c, palladium (Pd) diffuses from the second thin film activation layer 15s into the thin film layer 12 and promotes hydrogenation·dehydrogenation of the thin film layer 12. Thus, in the hydrogen sensor 10c, while the buffer layer 13 prevents the oxidation of the catalyst layer 14 and the thin film layer 12, and the second thin film activation layer 15s compensates for the decrease in hydrogen detection sensitivity attributed to the presence of the buffer layer 13. Preferably, in the present variation, the thickness of the second thin film activation layer 15s may be in the range of 1 to 5nm for the same reason as stated with respect to the buffer layer 13.

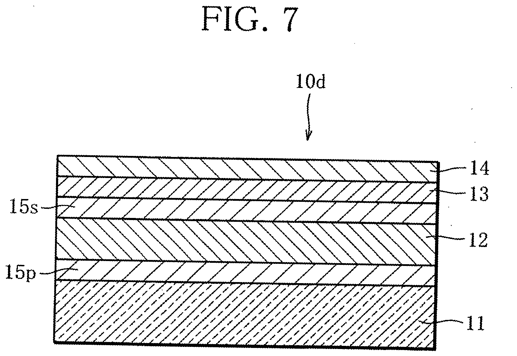

The hydrogen sensor 10d shown in FIG. 7 includes both a first thin film activation layer 15p, as included in the hydrogen sensor 10b according to FIG. 5 and a second thin film activation layer 15s, as included in the hydrogen sensor 10c according to the invention and as described in relation to FIG. 6 In this hydrogen sensor 10d, palladium (Pd) diffuses from the two thin film activation layers into the thin film layer 12. Thus, in this hydrogen sensor 10d, hydrogenation/dehydrogenation of the thin film layer 12 is more promoted, compared with the hydrogen sensors 10b and 10c. Consequently, the hydrogen sensor 10d has a further improved hydrogen detection sensitivity.

## Claims

1. An hydrogen sensor (10b), comprising:
a substrate (11);
a thin film layer (12) formed over the substrate (11);
a buffer layer (13) formed over the thin film layer (12); and
a catalyst layer (14) formed over the buffer layer (13), wherein said catalyst layer (14) when contacted by hydrogen gas in an atmosphere, hydrogenates the thin film layer (12), thereby changing optical reflectance of the thin film layer (12), **characterized in that**
the hydrogen sensor (10b) further comprises a thin film activation layer (15s) interposed between the buffer layer (13) and the thin film layer (12), wherein
the buffer layer (13) contains a constituent that diffuses into the catalyst layer (14) and combines with a constituent of the thin film layer (12) which diffuses from the thin film layer (12) into the buffer layer (13) and then into the catalyst layer (14), thereby restraining oxidation of the catalyst layer (14), and
the thin film activation layer (15s) contains a constituent which, when contacted by hydrogen, hydrogenates the thin film layer (12), thereby changing optical reflectance of the thin film layer (12).

2. The hydrogen sensor according to claim 1, wherein
the thin film layer (12) is formed of a magnesium alloy or magnesium, and
the catalyst layer (14) is formed to contain palladium or platinum.

3. The hydrogen sensor according to claim 2, wherein the thin film layer (12) is formed of a magnesium-nickel alloy, a magnesium-titanium alloy, a magnesium-niobium alloy, a magnesium-cobalt alloy or a magnesium-manganese alloy.

4. The hydrogen sensor according to claim 2, wherein the buffer layer (13) contains a constituent that combines with magnesium diffusing from the thin film layer (12) into the catalyst layer (14), thereby restraining the oxidation of the catalyst layer (14) attributed to the magnesium.

5. The hydrogen sensor according to claim 4, wherein the buffer layer (13) is formed to contain nickel, titanium, niobium or vanadium.

6. The hydrogen sensor according to claim 1, wherein the thickness of the buffer layer (13) is in a range of 1 to 5nm.

7. The hydrogen sensor according to claim 1, wherein the thin film activation layer (15s) is formed to contain the same constituent as the catalyst layer (14) contains.

8. The hydrogen sensor according to claim 7, wherein the thin film activation layer (15s) is formed to contain palladium or platinum.

9. The hydrogen sensor according to claim 1, wherein
the thin film layer (12) is formed over the substrate (11) with a first thin film activation layer (15p) interposed between the thin film layer (12) and the substrate (11), the buffer layer (13) is formed over the thin film layer (12) with a second thin film activation layer (15s) interposed between the buffer layer (13) and the thin film layer (12), and
the first and second thin film activation layers (15p, 15s) each contain a constituent which, by being contacted by hydrogen, hydrogenates the thin film layer (12), thereby changing optical reflectance of the thin film layer (12).

10. The hydrogen sensor according to claim 9, wherein the first and second thin film activation layers (15p, 15s) are formed to contain the same constituent as the catalyst layer (14) contains.

11. The hydrogen sensor according to claim 10, wherein the first and second thin film activation layers (15p, 15s) are formed to contain palladium or platinum.

## Patentansprüche

1. Wasserstoffsensor (10b), umfassend:
ein Substrat (11);
eine auf dem Substrat (11) ausgebildete Dünnfilmschicht (12);
eine auf der Dünnfilmschicht (12) ausgebildete Pufferschicht (13); und
eine auf der Pufferschicht (14) ausgebildete Katalysatorschicht (14), welche bei Kontakt mit Wasserstoffgas in einer Atmosphäre die Dünnfilmschicht (12) hydriert und dadurch die optische Reflektion der Dünnfilmschicht (12) verändert,
**dadurch gekennzeichnet,**
**dass** der Wasserstoffsensor (10b) des Weiteren eine zwischen der Pufferschicht (13) und der Dünnfilmschicht (12) angeordneten Dünnfilmschichtaktivierungsschicht (15s) aufweist, wobei
die Pufferschicht (13) einen Bestandteil beinhaltet, welcher sich in der Katalysatorschicht (14) ausbreitet und sich mit einem Bestandteil der Dünnfilmschicht (12) verbindet, welcher sich von der Dünnfilmschicht (12) in die Pufferschicht (13) und dann in die Katalysatorschicht (14) ausbreitet und dadurch eine Oxidation der Katalysatorschicht (14) hemmt, und
wobei die Dünnfilmaktivierungsschicht (15s) einen Bestandteil beinhaltet, welcher bei Kontaktierung mit Wasserstoff die Dünnfilmschicht (12) hydriert und dadurch die optische Reflektion der Dünnfilmschicht (12) verändert.

2. Wasserstoffsensor nach Anspruch 1, wobei
die Dünnfilmschicht (12) aus einer Magnesiumlegierung oder Magnesium ausgebildet ist und die Katalysatorschicht (14) zum Beinhalten von Palladium oder Platin ausgebildet ist.

3. Wasserstoffsensor nach Anspruch 2, wobei
die Dünnfilmschicht (12) aus einer Magnesium-Nickel-Legierung, einer Magnesium-Titan-Legierung, einer Magnesium-Niob-Legierung, einer Magnesium-Cobalt-Legierung oder einer Magnesium-Mangan-Legierung ausgebildet ist.

4. Wasserstoffsensor nach Anspruch 2, wobei
die Pufferschicht (13) einen Bestandteil beinhaltet, welcher sich mit von der Dünnfilmschicht (12) in die Katalysatorschicht (14) ausbreitendem Magnesium verbindet und dadurch die Oxidierung der dem Magnesium zugeordneten Katalysatorschicht (14) hemmt.

5. Wasserstoffsensor nach Anspruch 4, wobei
die Pufferschicht (13) zum Beinhalten von Nickel, Titan, Niob oder Vanadin ausgebildet ist.

6. Wasserstoffsensor nach Anspruch 1, wobei
die Dicke der Pufferschicht (13) in einem Bereich von 1 nm bis 5 nm liegt.

7. Wasserstoffsensor nach Anspruch 1, wobei
die Dünnfilmaktivierungsschicht (15s) zum Beinhalten der gleichen Bestandteile wie die Katalysatorschicht (14) ausgebildet ist.

8. Wasserstoffsensor nach Anspruch 7, wobei
die Dünnfilmaktivierungsschicht (15s) zum Beinhalten von Palladium oder Platin ausgebildet ist.

9. Wasserstoffsensor nach Anspruch 1, wobei
die Dünnfilmschicht (12) über dem Substrat (11) mit einer zwischen der Dünnfilmschicht (12) und dem Substrat (11) zwischengeordneten ersten Dünnfilmaktivierungsschicht (15p) ausgebildet ist, wobei die Pufferschicht (13) über der Dünnfilmschicht (12) mit einer zwischen der Pufferschicht (13) und der Dünnfilmschicht (12) zwischengeordneten zweiten Dünnfilmaktivierungsschicht (15s) ausgebildet ist, und
wobei die ersten und zweiten Dünnfilmaktivierungsschichten (15p, 15s) jeweils einen Bestandteil beinhalten, welcher durch Kontaktierung mit Wasserstoff die Dünnfilmschicht (12) hydriert und dadurch die optische Reflektion der Dünnfilmschicht (12) verändert.

10. Wasserstoffsensor nach Anspruch 9, wobei
die ersten und zweiten Dünnfilmaktivierungsschichten (15p, 15s) zum Beinhalten der gleichen Bestandteile wie die Katalysatorschicht (14) ausgebildet sind.

11. Wasserstoffsensor nach Anspruch 10, wobei
die ersten und zweiten Dünnfilmaktivierungsschichten (15p, 15s) zum Beinhalten von Palladium und Platin ausgebildet sind.

## Revendications

1. Capteur (10b) d'hydrogène, comprenant
un substrat (11) ;
une couche (12) de film mince formée sur le substrat (11) ;
une couche (13) de tampon formée sur la couche (12) de film mince ; et
une couche (14) de catalyseur formée sur la couche (13) de tampon, dans laquelle ladite couche (14) de catalyseur hydrogène la couche (12) de film mince quand ladite couche (14) de catalyseur est contactée par l'hydrogène par quoi la réflectance optique de la couche (12) de film mince est changée,
**caractérisé en ce que**
le capteur (10b) d'hydrogène comprend en outre une couche (15s) d'activation du film mince interposée entre la couche (13) de tampon et la couche (12) de film mince, dans lequel la couche (13) de tampon contient un constituant qui se diffuse dans la couche (14) de catalyseur et se combine avec un constituant de la couche (12) de film mince qui se diffuse de la couche (12) de film mince dans la couche (13) de tampon et après dans la couche (14) catalyseur par quoi l'oxydation de la couche (14) de catalyseur est retenue, et
la couche (15s) d'activation du film mince contient un constituant qui hydrogène la couche (12) de film mince quand ledit constituant est contacté par l'hydrogène par quoi la réflectance optique de la couche (12) de film mince est changée.

2. Capteur d'hydrogène selon la revendication 1, dans lequel la couche (12) de film mince est formée d'un alliage de magnésium ou du magnésium, et la couche (14) de catalyseur est formée à contenir du palladium ou du platine.

3. Capteur d'hydrogène selon la revendication 2, dans lequel la couche (12) de film mince est formée d'un alliage magnésium-nickel, d'un alliage magnésium-titane, d'un alliage magnésium-niobium, d'un alliage magnésium-cobalt ou d'un alliage magnésium-manganèse.

4. Capteur d'hydrogène selon la revendication 2, dans lequel la couche (13) de tampon contient un constituant qui se combine avec magnésium qui se diffuse de la couche (12) de film mince dans la couche (14) de catalyseur par quoi l'oxydation de la couche (14) de catalyseur attribuée au magnésium est retenue.

5. Capteur d'hydrogène selon la revendication 4, dans lequel la couche (13) de tampon est formée à contenir du nickel, du titane, du niobium ou du vanadium.

6. Capteur d'hydrogène selon la revendication 1, dans lequel l'épaisseur de la couche (13) de tampon est entre 1 nm et 5 nm.

7. Capteur d'hydrogène selon la revendication 1, dans lequel la couche (15s) d'activation du film mince est formée à contenir le même constituant que la couche (14) de catalyseur.

8. Capteur d'hydrogène selon la revendication 7, dans lequel la couche (15s) d'activation du film mince est formée à contenir du palladium ou du platine.

9. Capteur d'hydrogène selon la revendication 1, dans lequel la couche (12) de film mince est formée sur le substrat (11) ayant une première couche (15p) d'activation du film mince interposée entre la couche (12) de film mince et le substrat (11), la couche (13) de tampon étant formée sur la couche (12) de film mince ayant une deuxième couche (15s) d'activation du film mince interposée entre la couche (13) de tampon et la couche (12) de film mince, et
les premières et deuxièmes couches (15p, 15s) d'activation du film mince respectivement contiennent un constituant qui hydrogène la couche (12) de film mince en étant contacté par l'hydrogène par quoi la réflectance optique de la couche (12) de film mince est changée.

10. Capteur d'hydrogène selon la revendication 9, dans lequel les premières et deuxièmes couches (15p, 15s) d'activation du film mince sont formées à contenir le même constituant que la couche (14) de catalyseur.

11. Capteur d'hydrogène selon la revendication 10, dans lequel les premières et deuxièmes couches (15p, 15s) d'activation du film mince sont formées à contenir du palladium et du platine.
